# EUROPEAN PATENT SPECIFICATION

(11) **EP 3 957 718 B1**
(45) Date of publication and mention of the grant of the patent: **20.09.2023**
(21) Application number: 20208514.8
(22) Date of filing: 18.11.2020
(51) Int. Cl.: C12N 5/0775

(54) **METHOD FOR RESISTING AGING AND ENHANCING STEM CHARACTERISTICS OF HUMAN MESENCHYMAL STEM CELLS**
VERFAHREN ZUR ALTERUNGSBESTÄNDIGKEIT UND ZUR VERBESSERUNG DER STAMMEIGENSCHAFTEN VON MENSCHLICHEN MESENCHYMALEN STAMMZELLEN
PROCÉDÉ DE RÉSISTANCE AU VIEILLISSEMENT ET D'AMÉLIORATION DES CARACTÉRISTIQUES DE LA TIGE DE CELLULES SOUCHES MÉSENCHYMATEUSES HUMAINES

(30) Priority: 21.08.2020 CN 202010846598
(43) Date of publication of application: 23.02.2022
(73) Proprietor: Affiliated Hospital of Zunyi Medical University, Zunyi Guizhou (CN)
(72) Inventor: XIAO, Jianhui, Zunyi Guizhou (CN); LUO, Yi, Zunyi Guizhou (CN); ZHONG, Jianjiang, Zunyi Guizhou (CN); YU, Changyin, Zunyi Guizhou (CN)
(74) Representative: Ipey

(56) References cited:
- WO-A2-2014/011407
- ES-A1- 2 434 853
- US-A1- 2018 087 027
- ZHANG JIA-MIN ET AL: "Platelet-Derived Growth Factor-BB Protects Mesenchymal Stem Cells (MSCs) Derived From Immune Thrombocytopenia Patients Against Apoptosis and Senescence and Maintains MSC-Mediated Immunosuppression : PDGF-BB Corrects the Abnormalities in MSC-ITP", STEM CELLS TRANSLATIONAL MEDICINE, vol. 5, no. 12, 28 July 2016 (2016-07-28), pages 1631-1643, XP055797577, US ISSN: 2157-6564, DOI: 10.5966/sctm.2015-0360 Retrieved from the Internet: URL:https://api.wiley.com/onlinelibrary/td m/v1/articles/10.5966%2Fsctm.2015-0360>
- CHRISTINE FEHRER ET AL: "Reduced oxygen tension attenuates differentiation capacity of human mesenchymal stem cells and prolongs their lifespan", AGING CELL, BLACKWELL PUBLISHING, GB, vol. 6, no. 6, 1 December 2007 (2007-12-01), pages 745-757, XP002643661, ISSN: 1474-9718, DOI: 10.1111/J.1474-9726.2007.00336.X [retrieved on 2007-08-13]
- HYE JIN ET AL: "Comparative Analysis of Human Mesenchymal Stem Cells from Bone Marrow, Adipose Tissue, and Umbilical Cord Blood as Sources of Cell Therapy", INTERNATIONAL JOURNAL OF MOLECULAR SCIENCES, vol. 14, no. 9, 3 September 2013 (2013-09-03), pages 17986-18001, XP055202899, ISSN: 1661-6596, DOI: 10.3390/ijms140917986
- NIKETA SAREEN ET AL: "Early passaging of mesenchymal stem cells does not instigate significant modifications in their immunological behavior", STEM CELL RESEARCH & THERAPY, vol. 9, no. 1, 2 May 2018 (2018-05-02), XP055554291, DOI: 10.1186/s13287-018-0867-4
- SETHE S ET AL: "Aging of mesenchymal stem cells", AGEING RESEARCH REVIEWS, ELSEVIER, AMSTERDAM, NL, vol. 5, no. 1, 1 February 2006 (2006-02-01), pages 91-116, XP024959562, ISSN: 1568-1637, DOI: 10.1016/J.ARR.2005.10.001 [retrieved on 2006-02-01]
- GRUBER HE ET AL: "Human adipose-derived mesenchymal stem cells: serial passaging, doubling time and cell senescence", BIOTECHNIC AND HISTOCHEMISTRY., vol. 87, no. 4, 1 May 2012 (2012-05-01), pages 303-311, XP055797571, US ISSN: 1052-0295, DOI: 10.3109/10520295.2011.649785 Retrieved from the Internet: URL:https://www.tandfonline.com/doi/pdf/10 .3109/10520295.2011.649785?needAccess=true >
- Chinnadurai Raghavan ET AL: "Immune dysfunctionality of replicative senescent mesenchymal stromal cells is corrected by IFNg priming", Blood Advances, 25 April 2017 (2017-04-25), pages 628-643, XP055797574, Retrieved from the Internet: URL:https://watermark.silverchair.com/adva nces006205.pdf?token=AQECAHi208BE49Ooan9kk hW_Ercy7Dm3ZL_9Cf3qfKAc485ysgAAA9owggPWBgk qhkiG9w0BBwagggPHMIIDwwIBADCCA7wGCSqGSIb3D QEHATAeBglghkgBZQMEAS4wEQQMqsXgJn44SYTjpAw iAgEQgIIDjcX39PbQZGI50r__ZmrTYVHVmCFCKDK8J i3tj_dsFCEhxVM3BvTLNhsZDB-i9zWNBW97opSGubt PN1TWXEoEp [retrieved on 2021-04-21]
- LEGZDINA DIANA ET AL: "Characterization of Senescence of Culture-expanded Human Adipose-derived Mesenchymal Stem Cells", INTERNATIONAL JOURNAL OF STEM CELLS, vol. 9, no. 1, 31 May 2016 (2016-05-31), pages 124-136, XP055798171, ISSN: 2005-3606, DOI: 10.15283/ijsc.2016.9.1.124 Retrieved from the Internet: URL:https://www.ncbi.nlm.nih.gov/pmc/artic les/PMC4961112/pdf/ijsc-09-124.pdf>
- HU WANSHENG ET AL: "3D co-culture model of endothelial colony-forming cells (ECFCs) reverses late passage adipose-derived stem cell senescence for wound healing", STEM CELL RESEARCH & THERAPY, vol. 11, no. 1, 1 December 2020 (2020-12-01), XP055797578, DOI: 10.1186/s13287-020-01838-w Retrieved from the Internet: URL:https://stemcellres.biomedcentral.com/ track/pdf/10.1186/s13287-020-01838-w.pdf>
- MO A DAO ET AL: "Comparing the immunosuppressive potency of naïve marrow stromal cells and Notch-transfected marrow stromal cells", JOURNAL OF NEUROINFLAMMATION, BIOMED CENTRAL LTD., LONDON, GB, vol. 8, no. 1, 7 October 2011 (2011-10-07) , page 133, XP021112179, ISSN: 1742-2094, DOI: 10.1186/1742-2094-8-133

## Description

### FIELD OF THE APPLICATION

The present invention belongs to the field of stem cells and regenerative medicine, in particular, to a method for resisting aging and enhancing sternness characteristics of human mesenchymal stem cells in the long-term in vitro expansion process.

### BACKGROUND TECHNOLOGY

In recent years, mesenchymal stem cells (MSC) have become increasingly prominent in the treatment of human diseases due to their embryonic stem cell-like properties and paracrine capabilities, and have become an ideal cell source for stem cells and regenerative medicine (Arch Pharm Res. 2012;35:213- 21.). According to the records of the U.S. Clinical Trial Registry, there are more than a hundred kinds of diseases treated with MSC, especially for refractory diseases that show good application prospects (Stem Cells Transl Med 2019; 9:17-27.). However, the number of mesenchymal stem cells from human tissues is extremely limited, which is difficult to reach the minimum clinically required cell number: 10⁶-10⁷ cells per kilogram of body weight, and the adult dose is 10⁸-10⁹ per treatment (Cytotherapy 2018; 21:289-36). Therefore, in vitro expansion is the main way to solve the problem of insufficient number of MSCs (Nat Biomed Eng 2019; 3:90-104.). However, long-term in vitro culture causes a decrease in the expansion potential of MSCs and eventually leads to their division arrest (Arch Pharm Res. 2009; 32: 117-26); more importantly, MSC aging caused by the stress of amplification environment and age-related aging caused by long-term division and expansion of MSC lead to changes in cell expansion and differentiation gene proteins, further causing the progressive loss of excellent biological characteristics such as self-renewal, expansion ability, differentiation potential, immune regulation, migration and homing ability of the expanded MSC. Aging cells secrete a large number of aging-related secreted phenotypic factors that can cause the surrounding healthy cells to age. The infusion of aged MSC to patients directly affects the clinical therapeutic effect of stem cells, and even causes potential treatment risks such as immune rejection and causing healthy cells to become cancerous (Semin Immunol 2018, 40:101275 ; Trends Cell Biol 2018; 28:595-607). For example, aging reduces the expression of MSC cytokines and chemokine receptors (TNFR1, TNFR2, IL-6R), etc. (Nat Med. 2009;15:42-9.), so that their therapeutic effect in inflammatory diseases is much lower than that of young MSC (AmJRespir Crit Care Med. 2014;189:787-98). Therefore, the problem of cell aging caused by the long-term in vitro expansion of MSC is an important common scientific problem and main bottleneck that has not yet achieved large-scale clinical application of MSC, which is also a worldwide problem that needs to be solved urgently.

According to reports in the literature, MSC can regulate the microenvironment and the functions of surrounding cell by releasing various cytokines, growth factors and chemokines, and similarly, cells and extracellular matrix in the surrounding environment of MSC can also regulate the biological functions of MSC (Cell. 2018;175:908-920. Histol Histopathol. 2016;31:949-59). After the body tissue is damaged, the immune system is activated to limit the invasion of pathogens. At the same time, stem cells repair damage through expansion and differentiation to restore tissue integrity and homeostasis, and this process requires activating and coordinating the information exchange of the intracellular, the intercellular and the surrounding environment, wherein the immune system plays an important role in the process of damage repair (Cell Mol Life Sci. 2017;74:2345-60). In addition to recognizing and eliminating foreign antigens, the immune system can also monitor mutated tumor cells, aging cells and other harmful components in the body. In addition, the immune system also helps promote wound healing (Mediat Inflamm. 2016; 2016: 2856213.). After entering old age, the body's immune system is significantly degraded compared with young adults, and the ability to repair external damage is significantly reduced. Studies have shown that the thymus is a dual organ with both immune function and endocrine function, and an aging model can be constructed after removing the animal thymus (Mech Ageing Dev. 2001;122:1591-611.). Therefore, the decline of the immune system will cause the body to age. However, the stem cell aging hypothesis believes that the aging and depletion of adult stem cells are important causes of tissue and organ aging and aging-related diseases (Science 2007; 317:803-6); so, what is the connection between the aging of the body caused by the decline of the immune system and the aging of the body's adult stem cells? This scientific problem is still not completely clear. In normal tissues, adult stem cells are in a state of expansion arrest, but the adult stem cells in the damaged area are stimulated to repair damaged tissues through expansion and differentiation when the body is injured, with the process of tissue repair accompanied by the release of immune cells to the injured site; therefore, we speculate that immune cells play an important role in regulating the expansion and differentiation of local stem cells. Studies have found that natural lymphocytes stimulate intestinal stem cells by secreting IL-22 to regenerate and repair damaged intestinal epithelial cells (Nature. 2015;528:560-4). It is recently reported (Nature. 2019; 571:205-10) that the killer T cells expressing CD8 protein can break through the blood-brain barrier and infiltrate the brain of aging mice to interfere with the expansion and differentiation of neural stem cells. However, the regulatory effect of immune cells on aging stem cells has not yet been reported.

### Summary of Invention

An object of the present invention is to provide a safe and effective method for resisting ageing and enhancing stem characteristics of human MSCs in the long-term in vitro expansion process.

To achieve above object, the present invention comprises the method as defined in claim 1. The following documents are examples of disclosures co-culturing of immune cells with mesenchymal stem cells.

D1 ZHANG JIA-Min et al : "Platelet Derived Growth Factor-BB proptects mesenchymal stem Cells (MSCs) Derived from Immune Thrombocytopenia Patients against Apoptosis and Senescence and Maintains MSC_Mediated Immunosuppression: PDF Corrects the Abnormalities in MSC-ITP cells", STEM CELLS TRANSLATIONAL MEDICINE, vol. 5, no. 12, 28 July 2016, pages 1631 -1643 XP055797577 , ISSN: 2157-6564, DOI: 10.5966 /sctm.2015-0360.

D2 ES 234853 and WO2013/186418 (Fundacion Centro Nacional de Investigaciones Cardiovasculares Carlos III). This document relates to methods for determining the therapeutic potential and immunoregulatory capacity of stems cells, based on expression levels of microRNA miR-335. It also relates to in vitro methods for increasing the immunoregulatory capacity of a stem cell, in which the stem cell is treated with an miR-335 inhibitor.

D3 WO2014/011407 A2 (Imstem Biotechnology Inc) relates to methods of generating and expanding human embryonic stem cell derived mesenchymal-like stem/siromal cells having a low level of expression of IL-6.

None of the above shows the surprising effect achieved according to the invention, namely that of decreased β-galactosidase expression with a ratio of 100: 1 or above.

The immune cells from human peripheral blood used in the present invention are collected and separated from the donor population participating in the physical examination. The human peripheral blood immune cells include PBMC, or PBM in the PBMC, or PBL in the PBMC.

The human MSC used in the present invention may be obtained from fresh umbilical cord, amniotic membrane, and umbilical cord blood of healthy term cesarean section pregnant women, including amniotic membrane MSC, umbilical cord MSC and cord blood MSC.

In the method according to the present invention, the PBMC and the aged human MSCs are co-cultured in a cell-cell contact mode at a cell number ratio of 100:1 to 400:1. When the ratio of cells is more than 100 to 1, it shows a stronger reversal of the expression of β-galactosidase, a senescence marker of MSC.

In the method according to the present invention, the co-culturing PBMC from different donors aged 26-74 and the aged human MSCs in a cell-cell contact mode has no statistical difference in the expression of β-galactosidase, a marker of aging that reverses MSC.

In the method according to the present invention, the co-culturing three kinds of human peripheral blood immune cells and the aged human MSCs in a cell-cell contact mode includes a contact co-culture mode reverses the expression of β-galactosidase, a marker of aging in MSCs, and PBL has the best effect.

In the method according to the present invention, the co-culturing PBL and the young human MSCs or the aged human MSCs in a cell-cell contact mode may delay or reverse the aging of human MSCs, and significantly reduce the expression of cell aging markers β-galactosidase, P16 and P21.

In the method according to the present invention, the co-culturing PBL and the aged human MSCs in a cell-cell contact mode may regulate the cell cycle of aging MSCs, reduce the proportion of cells in G1 phase, and increase the proportion of cells in S phase.

In the method according to the present invention, the PBL and the aged human MSCs are co-cultured in a cell-cell contact way, which may significantly improve the proliferation ability of the MSC, and promote the expression of the proliferating cell nuclear antigen PCNA.

In the method according to the present invention, the PBL and the aged human MSCs are co-cultured in a cell-cell contact way, which may improve the self-renewal ability of MSC, and significantly promote the formation of human MSC clones by more than 10 times.

In the method according to the present invention, the PBMC and the aged human MSCs are co-cultured in a cell-cell contact way, which may significantly enhance the expression of Oct4, a stem characteristic marker of MSCs, and improve the multi-differentiation ability of human MSCs, including differentiation into osteoblasts, chondrocytes and adipocytes.

In the method according to the present invention, the PBL and the aged human MSCs are co-cultured in a cell-cell contact way for expansion to obtain the MSC, which is used to treat animal models of diseases, without immune elimination reaction, which is safe and effective.

In the present invention, the aged (age-related senescence) human MSC is the research object, and co-cultured with immune cells in human peripheral blood including peripheral blood mononuclear cells (PBMC) and peripheral blood monocytes (PBM) and peripheral blood lymphocytes (PBL) in PBMC in a cell-cell contact or non-contact way; it is found that the method may significantly reverse the aging characteristics of aged human MSCs, delay the age-related aging of non-aged human MSCs, and reduce the expression of β-galactosidase, P16 and P21 and other cellular aging markers. The method may regulate the cell cycle of aging MSCs, reduce the proportion of cells in G1 phase, and increase the proportion of cells in S phase. More importantly, the method can significantly enhance the stem characteristics of the aging human mesenchymal stem cells, such as abilities of self-renewal, proliferation and multidirectional differentiation potential. The human peripheral blood immune cells PBL and the aged human MSCs are co-cultured and expanded by the cell-cell contact way, resulting in the MSC that is used for the treatment of disease models without immune rejection and with significant therapeutic effect. The method has great application value to solve the bottleneck problem of aging and proliferative aging caused by environmental stress and oxidative stress damage in the long-term in vitro expansion of stem cells.

### Brief Description of Drawings

Fig. 1 illustrates the phenotype identification of human MSC (taking human amniotic membrane MSC as an example). (A) flow cytometry to detect surface molecular markers; (B) immunocytochemical staining to detect vimentin and keratin CK19.
Fig. 2 shows the changes of the number of β - galactosidase positive cells (aging cells) after PBMC with different cell ratios are co-cultured with aged MSC.
Fig. 3 shows the changes of the number of β - galactosidase positive cells (aged cells) after PBMC and aged MSC are co-cultured in different ways (contact and non-contact).
Fig. 4 shows the changes of the number of β - galactosidase positive cells (aged cells) after PBMC from different donor ages are co-cultured with aged MSC.
Fig. 5 shows the changes of the number of β - galactosidase positive cells (aged cells) after different immune cells in human peripheral blood are co-cultured with aged MSC.
Fig. 6 shows the changes of the number of β - galactosidase positive cells (aged cells) after PBL are co-cultured with human umbilical cord mesenchymal stem cells.
Fig. 7 shows the changes of the number of β - galactosidase positive cells (aged cells) after PBL are co-cultured with human umbilical cord blood mesenchymal stem cells.
Fig. 8 shows the changes in the expression of the aged markers P21 and P16 proteins in the aged MSC detected by western blotting after PBL is contacted and co-cultured with the aged MSC.
Fig. 9 shows the flow cytometry analysis of the cell cycle changes of the aged MSC after the PBL is contacted and co-cultured with the aged MSC.
Fig. 10 shows the changes in the expansion ability of the aged MSCs detected by the EDU kit after the PBL is co-cultured with the aged MSCs.
Fig. 11 shows the cloning ability experiment of aged MSCs after 15 days of co-culturing PBL with aged MSCs.
Fig. 12 shows the changes in the expression of proliferating cell nuclear antigen PCNA and the stem transcription factor Oct4 in the aged MSCs detected by western blotting after PBL is contacted and co-cultured with aged MSCs.
Fig. 13 shows the changes in the ability of aged MSCs to differentiate into osteogenic, cartilage and adipocytes after PBL is co-cultured with aged MSC.
Fig. 14 shows the effect of β-galactosidase staining on delaying MSC aging after long-term contact of PBL and non-aged MSC for 12 days.
Fig. 15 shows the levels in the expression of the aged markers P21 and P16 proteins in the MSC detected by western blotting after PBL is contacted and co-cultured with the non-aged MSC for a long time of 12 days.
Fig. 16 shows the efficacy evaluation of MSCs obtained after PBL and aged MSCs are contacted and co-cultured on ulcerative colitis mouse models. (A) mouse body weight change; (B) mouse disease activity index (DAI) score; (C) pathological observation; (D) pathological score

### Examples

In order to more fully explain the implementation of the present invention, and that the objectives, technical schemes and advantages of the present invention will become more apparent, the technical solutions of the present invention will be described in more detail with reference to the drawings and examples above. MSCs from different tissues have no effect on the results (see Embodiment 7). The present embodiment mainly uses human amniotic membrane MSCs for illustration. It should be understood that the specific embodiments described herein are only for illustrating but not for limiting the present invention. In addition, the embodiment of the present invention passed the review of the ethics committee of the Affiliated Hospital of Zunyi Medical College (ethics review number: KLLY-2017-003).

### Example 1: Isolation, culture and identification of human mesenchymal stem cells

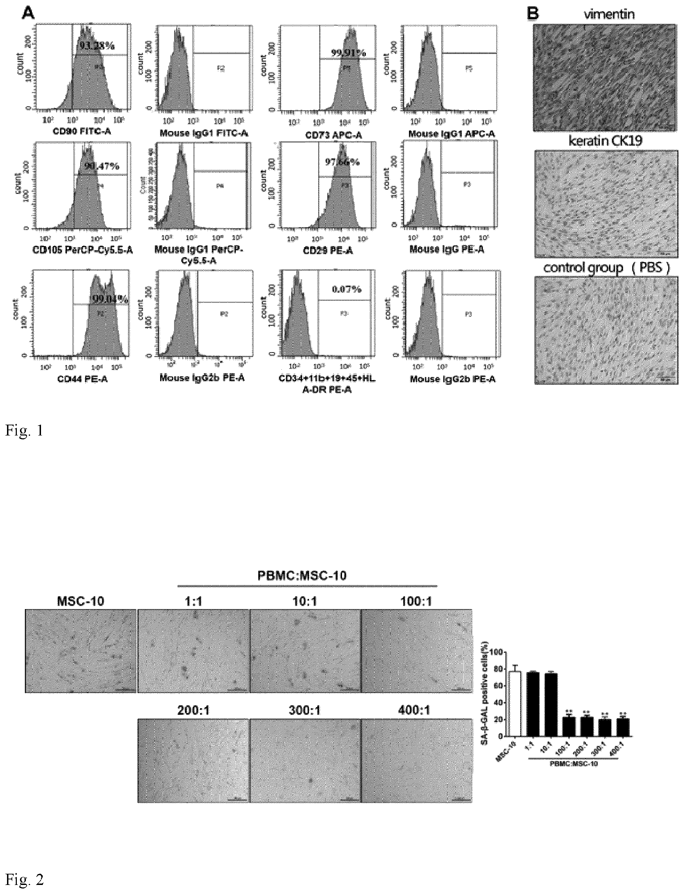

The amniotic membrane tissue is stripped from the fresh placenta of healthy term cesarean section, and the residual blood stains and mucus are repeatedly washed with D-PBS solution containing 1% bi-antibody (final concentration including penicillin of 100 IU/mL, and streptomycin of 100 IU/mL; freshly prepared before use). After cutting the amniotic membrane to a size of about 1 cm², it is divided into 50 mL centrifuge tubes, and then 0.05% trypsin digestion solution containing 0.02% EDTA-2Na that is about 2 times the volume of the amniotic membrane tissue is added for digesting in a constant temperature water bath at 37 °C at 185 rpm for about 30 minutes, filtering with 300 mesh stainless steel filter, and removing the supernatant; then, the above steps are repeated. The digested amniotic membrane is washed with D-PBS containing 1% bi-antibody once, and an equal volume of 0.5 mg/mL type II collagenase digestion solution containing 0.05 mg/mL DNase I is added for rotating and digesting at 190 rpm at 37 °C for 1 to 1.5 h until the amniotic membrane fragments are completely digested into flocculent, followed by filtering with a 300 mesh filter and collecting cell filtrate for centrifuging at 1500 rpm for 10 minutes; then, the supernatant is removed, and the cell pellet is the human amnion-derived mesenchymal stem cells (hA-MSC). The cells are resuspended in low-glucose DMEM complete medium containing 10% FBS, plated in 75-cm² cell culture flasks, cultured at a constant temperature with 5% CO₂ and 85-100% air saturated humidity at 37 °C, and subcultured when the confluence of the cells reaches 80% or more, so as to collect the passage 3 (P3) or passage 9 (P9) cells for experiments. Further, the human umbilical cord-derived mesenchymal stem cells (human umbilical cord-derived mesenchymal stem cells, hUC-MSC) are separated from fresh umbilical cord tissue using the tissue block adhesion method, and the human umbilical cord blood-derived mesenchymal stem cells (human umbilical cord blood-derived mesenchymal stem cells, hUCB-MSC) are separated from fresh cord blood by the density gradient centrifugation; all are purified by trypsin, and the P3 cells are collected for experiments. These mesenchymal stem cells highly express CD90, CD105, CD73, CD44 and CD29 and other mesenchymal cell surface molecules, do not express hematopoietic stem cell markers such as CD34, CD11b, CD19, CD45 and HLA-DR and MHC class II cell surface molecules (Fig. 1A). The results of immunocytochemical staining show that the cells highly express vimentin, a mesenchymal cell marker, but do not express cytokeratin 19 (CK19), a marker of epithelial cells (Fig. 1B). They have a typical mesenchymal cell phenotype.

### Example 2: Isolation of human peripheral blood immune cells

Fresh peripheral blood from a normal physical examination population is taken for diluting it with an equal volume of sterile D-PBS. An appropriate amount of Histopaque-1077 is added to a 15 mL centrifuge tube, and an equal volume of sterile D-PBS diluted blood is slowly added along the tube wall for centrifuging at 2000 rpm for 20min; the middle albuginea layer is extracted, and an equal volume of sterile D-PBS is added for centrifuging at 1500 rpm for 10 min; washing is performed repeatedly with sterile D-PBS once, and the supernatant is discarded; the cell pellet is suspended in DMEM medium containing 10% FBS and low glucose to obtain PBMC immune cells. With the characteristics of peripheral blood mononuclear cells (PBM) that are easy to adhere to and grow on plastic cell culture plates, the PBM and peripheral blood lymphocytes (PBL) are separated from the above PBMC.

### Example 3: Comparison the reversal effects on aged cells of contact co-culturing PBMC with the aged MSC at different cell ratios

The human amniotic membrane MSCs that are continuously expanded to the P9 in vitro and have replicative aging is taken for inoculating on a 12-well cell culture plate at a density of 10⁴/well, and the freshly-isolated PBMC is added after 16 h, wherein the density of PBMC is 10⁴, 10⁵, 10⁶, 2×10⁶, 3×10⁶ and 4×10⁶/well respectively, and the PBMC and the MSC are co-cultured at cell ratios of 1:1, 10:1, 100:1, 200:1, 300:1 and 400:1. After PBMC and MSC are co-cultured for 72 hours, β-galactosidase staining is used to detect the number of positive aged MSCs. The results (Fig. 2) show that the rate of β-galactosidase positive cells in the control group of aged MSCs cultured to the 10th generation (hereinafter referred to as "MSC-10") is (77.07±7.23)%, and when PBMC:MSC-10=1:1 or 10:1, the positive rate of β-galactosidase does not change significantly as compared with the aging control group; when PBMC:MSC-10 reaches 100:1, the positive rate of β-galactosidase begins to decrease significantly, from 77.07% to 22.61%, and when the cell ratio of co-culturing PBMC with aged MSC continues to increase, the positive rate of β-galactosidase still shows a downward trend as compared with the control group; the effect is best when the cell ratio is 300:1, and the rate of β-galactosidase positive cells is only (20.07±3.07)% (Table 1). Therefore, when PBMC and MSC are co-cultured at a cell ratio of 100:1-400:1, the aged MSC may be significantly rejuvenated, showing the effect of reversing aging.

**Table 1 is β-galactosidase staining to detect the reversal effect on aged cells of PBMC and aged MSC contact co-culturing at different cell ratios.**

| cell ratios in co-culturing rate of β-galactosidase positive (PBMC:MSC-10) | cells (%) |
|---|---|
| 1:1 | 75.8±1.47 |
| 10:1 | 74.19±2.94 |
| 100:1 | 22.61±3.26** |
| 200:1 | 22.58±2.27** |
| 300:1 | 20.07±3.07** |
| 400:1 | 20.92±2.55** |

| | |
|---|---|
| Note: The rate of β-galactosidase positive cells in the MSC-10 group is (77.07±7.23)%, and compared with MSC-10, ^{∗∗}*p*<0.01. | |

### Example 4: Comparison the reversal effects on aged cells of contact and non-contact co-culturing PBMC and the aged MSC

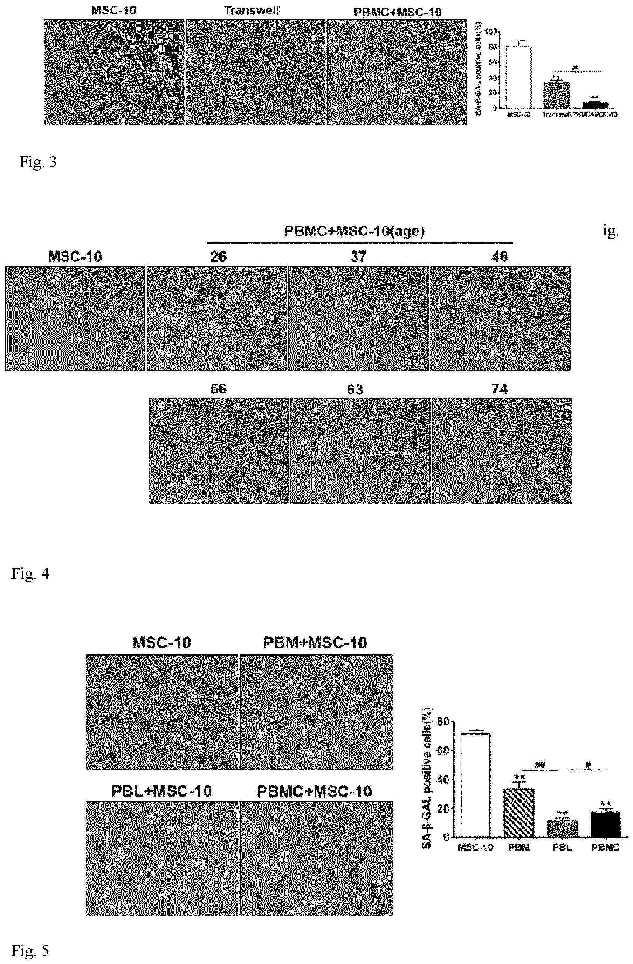

The human amniotic membrane MSCs that are continuously expanded to the P9 in vitro and have replicative aging is taken for inoculating into the lower chamber of Transwell (Corning, 3401) at a density of 10⁴/well, and adding10⁶ freshly-separated PBMCs in the lower or upper chambers respectively to be co-cultured with MSCs; after 72 h, the cells in the lower chamber are observed and β-galactosidase staining is performed to detect aged positive cells. The results (Fig. 3) show that both contact and non-contact co-culturing of PBMC and MSC may significantly reduce the positive staining rate of β-galactosidase in aged MSCs, make aged cells younger, and reverse aging. The effect in reversing of the contact co-culturing is better than that of the non-contact co-culturing.

### Example 5: Comparison the reversal effects on aged cells of contact co-culturing PBMC from different donor ages and aged MSC

The human amniotic membrane MSCs that are continuously expanded to the P9 in vitro and have replicative aging is taken for inoculating on a 12-well cell culture plate at a density of 10⁴/well, and freshly-separated PBMCs from different age groups are added after 16 h. After aged MSC (MSC-10) and PBMC are co-cultured for 72 h, the aged positive cells are detected by β-galactosidase staining. The results (Fig. 4) show that the positive rate of β-galactosidase staining in the aged MSC group is (69.75±4.79)%; compared with the above, PBMC from different donor ages may significantly reduce the positive rate of β-galactosidase staining in the co-culture system when added to the aged MSC group, rejuvenating aged cells with effects in reversing aging. However, the mean value of the rate of β-galactosidase staining positive cells after co-culturing PBMCs from donors of different ages and aged MSCs ranges from (19.56±4.20)% to (20.84±4.35)% (Table 2), and there is no statistical difference between the groups in the effects in reversing aging.

**Table 2 is β-galactosidase staining to detect the reversal effect on aged cells of contact co-culturing PBMC from different donor ages and the aged MSC .**

| age group of immune cell number of rate of β-galactosidase positive donor (years) | cases | cells (%) |
|---|---|---|
| | | |
| 21-30 | 10 | 19.90±2.71** |
| 31-40 | 10 | 20.84±4.35** |
| 41-50 | 10 | 19.56±4.20** |
| 51-60 | 10 | 20.35±5.48** |
| 61-70 | 10 | 20.74±4.26** |
| 71-80 | 10 | 22.27±3.36** |

| | | |
|---|---|---|
| Note: The rate of β-galactosidase positive cells in the MSC-10 group is (69.75±4.79)%, and compared with MSC-10, ^{∗∗}*p*<0.01. | | |

### Example 6: Comparison the reversal effects on aged cells of contact co-culturing different immune cells from human peripheral blood and aged MSC

Density gradient centrifugation is used to separate PBMC from the peripheral blood of the physical examination population, and seeded on a 12-well cell culture plate at a density of 2×10⁶/well; after 2 h, with the characteristics of PBM that it is easy to adhere to the plastic culture plate to grow on the wall, the PBM and the PBL are separated from the PBMC. Further, the human amniotic membrane MSCs that are continuously expanded to the P9 in vitro and have replicative aging is taken for inoculating respectively on 12-well cell culture plates containing PBMC, PBM and PBL at a density of 10⁴/well, and β-galactosidase staining is used to detect the effects of PBMC, PBM and PBL on aged MSCs in the co-culture system after 72 h of co-culturing. The results (Fig. 5) show that co-culturing PBMC, PBM and PBL with aged MSC may reduce the positive rate of β-galactosidase staining (aged cells), make aged cells younger, and have the effect of reversing aging; the rate of β-galactosidase positive cells in the aged cell MSC-10 group is (71.60±2.49)%, and the rates of β-galactosidase positive cells measured by co-culturing PBMC, PBM and PBL with the aged cell MSC-10 group are (17.52±2.24)%, (33.67±4.77)% and (11.39±2.17)% (Table 3), i.e., the effect of these immune cells in reversing aging is: PBL>PBMC>PBM.

**Table 3 is β-galactosidase staining to detect the reversal effect on aged cells of different immune cells and aged MSC contact co-culturing.**

| Groups | rate of β-galactosidase positive cells (%) |
|---|---|
| MSC-10 | 71.60±2.49 |
| PBM+MSC-10 | 33.67±4.77** |
| PBL+MSC-10 | 11.39±2.17**^{#&} |
| PBMC+MSC-10 | 17.52±2.24**^{##} |

| | |
|---|---|
| Note: Compared with MSC-10, ^{∗∗}*p*<0.01; compared with PBM+MSC-10, ^{##}*p*<0.01; compared with PBMC+MSC-10, *^{&}p*<0.05. | |

### Example 7: Comparison the reversal effects on aged cells of contact co-culturing PBL and aged MSCs derived from umbilical cord and cord blood

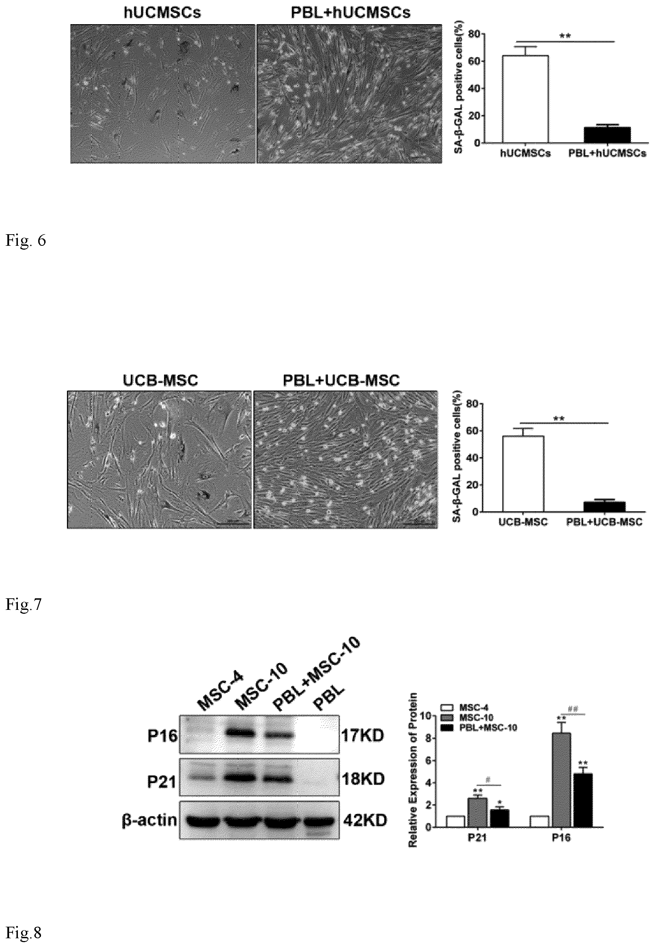

The human umbilical cord-derived mesenchymal stem cells (hUC-MSC) are separated from fresh umbilical cord tissue using the tissue block adhesion method, and the human umbilical cord blood-derived mesenchymal stem cells (hUCB-MSC) are separated from fresh cord blood by the density gradient centrifugation; all are purified by trypsin, and the P9 cells are collected for experiments. The P9 hUC-MSC and hUCB-MSC with replicative aging are taken for inoculating in a 12-well cell culture plate at a density of 10⁴/well, and the freshly-separated PBL is added after 16 h for co-culturing for 72 h; then, β-galactosidase staining is used to detect the ability of PBL to reverse the aging characteristics of aged hUC-MSC and aged hUCB-MSC. The results show that similar to the results of contact co-culturing PBL and aged human amniotic membrane MSC, the contact co-culture of PBL and the aged hUCMSC may significantly reverse the aging characteristics of aged hUC-MSC (Fig. 6); similarly, the contact co-culture of PBL and the aged hUCB-MSC may also significantly reverse the aging characteristics of aged hUCB-MSC (Fig. 7). Therefore, the effect of PBL in reversing the aging characteristics of aged human MSC is not limited to the tissue source of MSC. It has the effect of reversing aging and rejuvenating other solid tissues including blood-derived MSC. The following, with the human amniotic membrane MSC as an example, describes the effects of co-culturing PBL in reversing the aging of the aged MSC and delaying long-time in vitro expansion aging of the non-aged MSC, while evaluating the biological characteristics and functions.

### Example 8: Contact co-culturing PBL and aged MSC may reduce the expression of aged marker proteins P21 and P16.

The MSCs that have been continuously expanded to the P9 in vitro and have replicative aging are taken for inoculating in a cell culture dish with a diameter of 10 cm at a density of 2×10⁵, and the freshly-separately PBL is added after 16 h for co-culturing for 72 h; then the total cell protein is extracted, and western blotting is used to analyze the expression of aged markers P21 and P16 proteins. The results (Fig. 8) show that co-culturing PBL and aged MSC may significantly reduce the expression of aged marker proteins P21 and P16 of MSC in the co-culture system.

### Example 9: Contact co-culturing PBL and aged MSC may regulate the cell cycle of aging MSC.

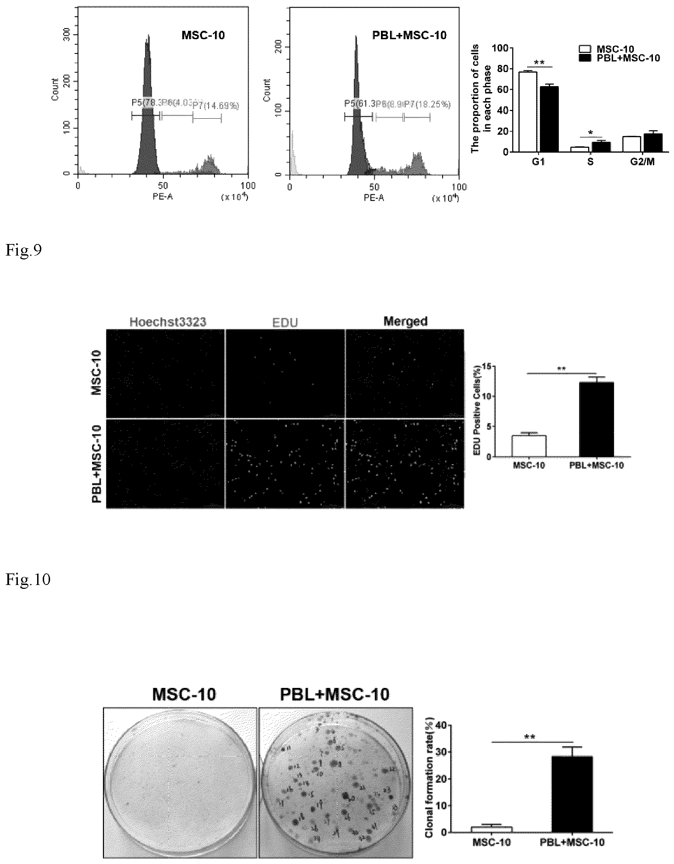

The MSCs that have been continuously expanded to the P9 in vitro and have replicative aging are taken for inoculating in a cell culture dish with a diameter of 10 cm at a density of 2×10⁵, and the freshly-separately PBL is added after 16 h for co-culturing for 72 h; then the cells are washed 6 times with sterile D-PBS, trypsinized and then centrifuged at low speed to collect the cells, and the DNA content detection kit (Solarbio, CA1510) is used to detect the cell cycle. The results (Fig. 9) show that co-culturing PBL and aged MSC may significantly regulate the cell cycle of MSC in the co-culture system, specifically represented by significantly reducing the cell ratio in G1 phase and increasing the cell ratio in S phase, suggesting that aged MSC cells restart division and proliferation.

### Example 10: Contact co-culturing PBL and aged MSC may reshape the expansion ability and self-renewal ability of aged MSC.

The MSCs that are continuously expanded to the P9 in vitro and have replicative aging is taken for inoculating on 24-well cell culture plate at a density of 3×103/well, and after 16 h, freshly isolated PBL is added and co-cultured for 72 h; then, EDU kit is used to detect the effect of PBL on the expansion of aged MSC. The results (Fig. 10) show that after PBL and aged MSC are co-cultured, the EDU positive cell rate increases from 3.54% to 12.31% (Table 4), which significantly improves the expansion ability of aged MSC.

**Table 4 shows reshaping the expansion ability of aged MSC detected by EDU staining in case where PBL and aged MSC are co-cultured in the contact way.**

| Groups | EDU positive cell rate (%) |
|---|---|
| MSC-10 | 3.54±0.47 |
| PBL+MSC-10 | 12.30±0.91** |

| | |
|---|---|
| Note: Compared with MSC-10, ^{∗∗}*p*<0.01. | |

### Example 11: Contact co-culturing PBL and aged MSC may enhance the cloning ability of aged MSC.

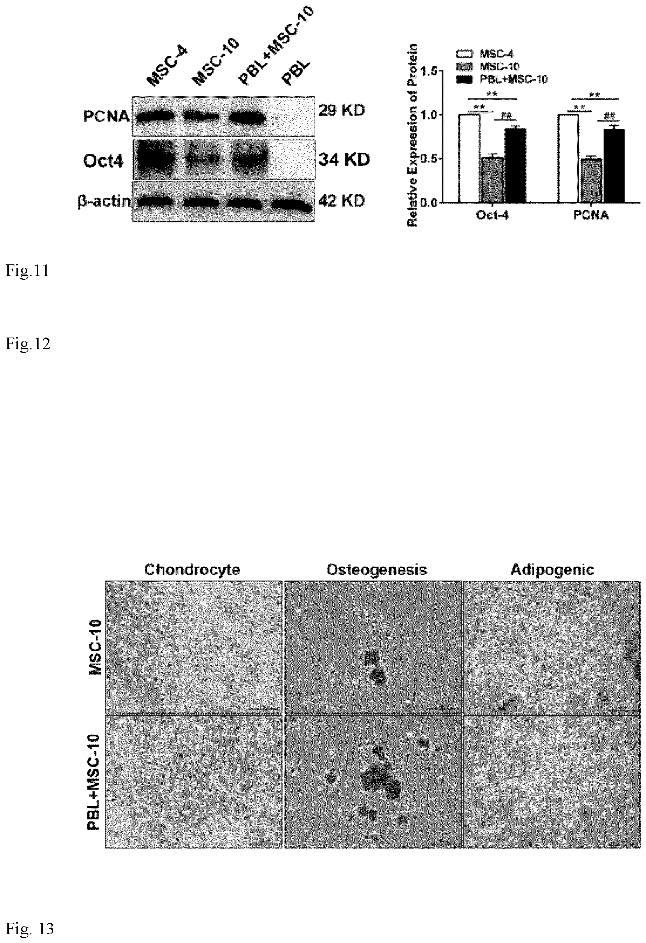

The MSCs that are continuously expanded to the P9 in vitro and have replicative aging is taken for seeding in a cell culture dish with a diameter of 10 cm at a density of 200 cells per dish, and after 16 h, freshly isolated PBL is added and the medium is changed every 6 days; after 15 days of co-culturing, the cell colony formation of aged MSCs is observed by crystal violet staining and recorded by taking pictures. The results (Fig. 11) show that after 15 days of co-culturing PBL and aged MSC, a significant increase in cell colonies is observed, and the colony formation rate increases by more than 10 times (Table 5). Therefore, this method may reshape the self-renewal ability of aged MSC.

**Table 5 shows enhancement of the cloning ability of aged MSC detected by crystal violet staining in case where PBL and aged MSC are co-cultured in a contact way.**

| Groups | clone formation rate (%) |
|---|---|
| MSC-10 | 1.33±0.58 |
| PBL+MSC-10 | 28.33±3.51** |

| | |
|---|---|
| Note: Compared with MSC-10, ^{∗∗}*p*<0.01. | |

### Example 12: Contact co-culturing PBL and aged MSC may enhance the expression of PCNA and stem transcription factor Oct4 in aged MSC.

The MSCs that have been continuously expanded to the P9 in vitro and have replicative aging are taken for inoculating in a cell culture dish with a diameter of 10 cm at a density of 2×10⁵, and the freshly-separately PBL is added after 16 h for co-culturing for 72 h; then the total cell protein is extracted, and Western blotting is used to analyze the expression of proliferating cell nuclear antigen PCNA and stem transcription factor Oct4. The results (Fig. 12) show that after PBL and the aged MSC are co-cultured, the expression level of the proliferating cell nuclear antigen PCNA and the sternness characteristic transcription factor Oct4 in aged MSC may be significantly enhanced.

### Example 13: Contact co-culturing PBL and aged MSC may improve the multi-directional differentiation ability of aged MSC.

The MSCs that are continuously expanded to the P9 in vitro and have replicative aging is taken for inoculating in 6-well cell culture plate at a density of 2×10⁴/well, and after 16 h, freshly isolated PBL is added and co-cultured for 72 hours; then, when the cell confluence reaches 80%, the medium is replaced with osteogenic and chondrogenic differentiation medium, and when the cell confluence reaches 100%, the medium is replaced with adipogenic differentiation medium. During the whole process of induction and differentiation, the medium is changed every 3 days, and on 21th day, toluidine blue staining is used to detect the production of glycosaminoglycans in the extracellular matrix of chondrogenic differentiation, alizarin red S staining is used to determine the formation of osteogenic differentiation calcified nodules, and oil red O staining is used to determine the formation of adipogenic lipid droplets. The results (Fig. 13) show that Toluidine Blue staining detects that the aged MSC after PBL treatment had a strong ability to produce glycosaminoglycans in cartilage matrix; Alizarin Red S staining shows stronger calcium nodule formation ability; Oil Red O staining shows more bright red lipid droplets are formed. Therefore, the method has the ability to enhance the differentiation ability of aged MSCs into osteogenic, adipogenic and chondrogenic.

### Example 14: Long-term co-culturing PBL and non-aged MSC may delay MSC aging.

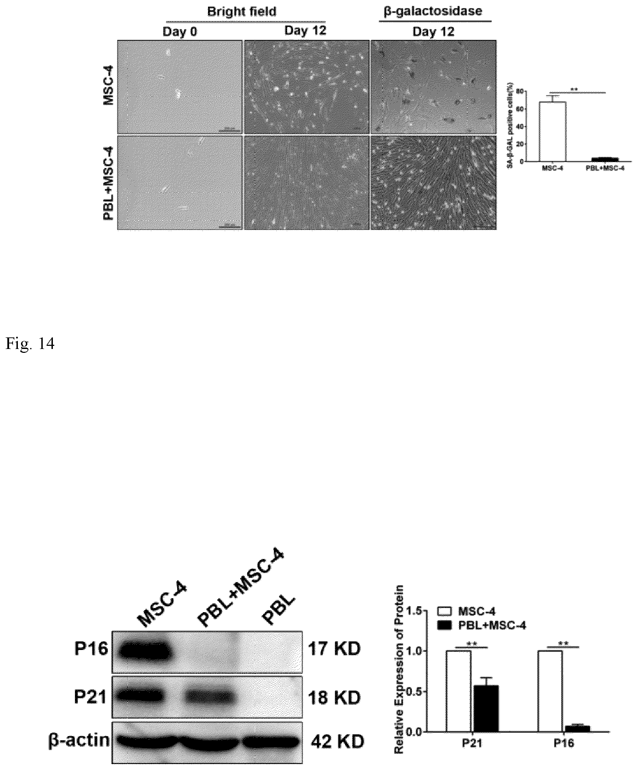

The P3 MSC in the logarithmic growth phase is taken for inoculating in a 6-well cell culture plate at a density of 10³/well; after 16 h, freshly separated PBL is added, and the liquid is changed every 4 days; after continuous culture for 12 days, the cells are taken out, and aging positive cells are detected by β-galactosidase staining. The results (Fig. 14) show that after 12 days of continuous in vitro culture, the MSCs appear flat and wide in cell bodies, and show a positive staining rate of β-galactosidase of 68.5%, suggesting that the long-term in vitro expansion and culture will lead to the aging of hAMSCs. However, after 12 days of co-culturing PBL and MSC, the cell morphology still shows a long spindle-shaped spiral growth, and the positive staining rate of β-galactosidase is only 3.9%. Therefore, the method may significantly delay the occurrence of MSC aging during long-term in vitro expansion.

### Example 15: Long-term contact co-culturing PBL and non-aged MSC may significantly reduce the expression of aged marker proteins P21 and P16 in MSC.

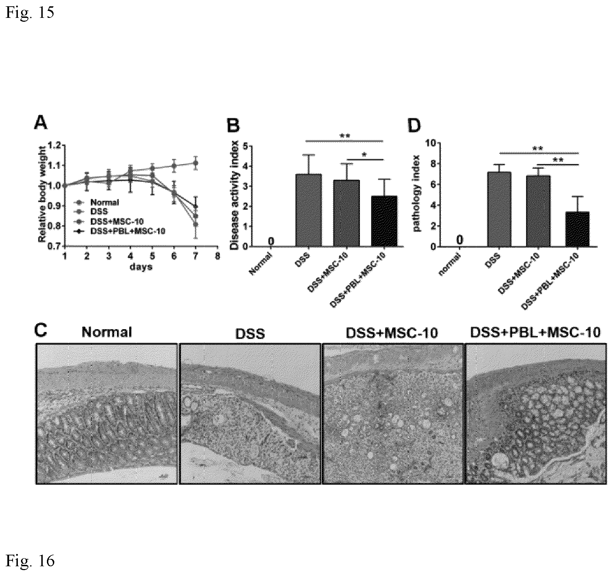

The P3 of MSCs in the logarithmic growth phase are taken for inoculating in a cell culture dish with a diameter of 10 cm at a density of 10⁴, and the freshly-separately PBL is added after 16 h; the medium is changed every 4 days, and after 12 days of continuous culture, the total cell protein is extracted, and western blotting is used to analyze the expression of aged marker proteins P21 and P16. The results (Fig. 15) show that after PBL and MSC are co-cultured for 12 days, the expression levels of aged marker proteins P21 and P16 of MSC are significantly lower than those in the non-co-culture group.

### Example 16: Therapeutic effect of MSC obtained by expansion with contact co-culturing PBL and aged MSC on disease models

A mouse model of ulcerative colitis is constructed by free diet 3% DSS, and C57 mice aged 5-7 weeks are randomly divided into 4 groups, a total of 40 mice, 10 mice in each group, which are marked with Normal, DSS, DSS+MSC-10 and DSS+PBL+MSC-10 respectively. The DSS+MSC-10 group and the DSS+PBL+MSC-10 group are treated with P10 and lymphocytes by intraperitoneal injection of P10 MSCs (1×10⁷/head) after 72 hours at the start of feeding DSS. Normal and DSS mice are injected with equal volume of sterile PBS at the same time. During the observation, the weight is regularly measured and the death situation is recorded every day, as well as observing whether there are loose stools, bloody stools, etc. The disease activity index (DAI) is calculated based on the weight, diarrhea, blood in the stool, and death of the mouse. Ten days after injection of MSC, all the mice are sacrificed, and the colon segment is fixed with 4% paraformaldehyde overnight at room temperature, embedded in paraffin, and cut into 4 µm sections for HE staining. The inflammatory cell infiltration and crypt and goblet cell structure of colon tissue are observed under a microscope, and histopathological score is performed. The results show that on the 5th day of feeding DSS, the mice begin to show severe inflammatory colitis symptoms, including weight loss, loose stools, bloody stools, etc., and die on the 7th day (Fig. 16); histological analysis shows that DSS feeding causes severe inflammation and damage to the colonic mucosa of mice (Figs. 16C and D). The therapeutic effect of P10 MSC on mice with ulcerative colitis is similar to that of DSS group, wherein there is severe weight loss and bloody stools, and histopathological observation of the colon shows severe inflammatory cell infiltration and loss of crypt and goblet cell structure. However, injecting P10 MSCs obtained by expansion with co-culturing PBL and aged MSCs may significantly reduce the weight loss, bloody stools, colon inflammation and damage induced by DSS in mice (Fig. 16), without immune rejection and causing death, with the obvious therapeutic effect.

## Claims

1. A method for resisting ageing and enhancing sternness characteristics of human mesenchymal stem cells *in vitro,* comprising co-culturing immune cells derived from human peripheral blood comprising human peripheral blood lymphocytes, with human mesenchymal stem cells, the co-culturing being in contact mode at a cell number ratio of the human peripheral blood lymphocytes to the human mesenchymal stem cells of 100 to 400:1.

## Patentansprüche

1. Verfahren zur Alterungsbeständigkeit und Verbesserung der Stammeigenschaften von menschlichen mesenchymalen Stammzellen *in vitro,* umfassend Co-Kultivieren von Immunzellen, die von menschlichem peripheren Blut abgeleitet sind, das Lymphozyten des menschlichen peripheren Bluts umfasst, mit menschlichen mesenchymalen Stammzellen, wobei das Co-Kultivieren in einem Kontaktmodus in einem Zellzahlverhältnis der Lymphozyten des menschlichen peripheren Bluts zu den menschlichen mesenchymalen Stammzellen von 100 bis 400:1 erfolgt.

## Revendications

1. Méthode pour résister au vieillissement et améliorer les caractéristiques de souche de cellules souches mésenchymateuses humaines *in vitro,* comprenant la co-culture de cellules immunitaires dérivées du sang périphérique humain, comprenant des lymphocytes du sang périphérique humain, avec des cellules souches mésenchymateuses humaines, la co-culture étant en mode contact à un rapport de nombre de cellules entre les lymphocytes du sang périphérique humain et les cellules souches mésenchymateuses humaines de 100 à 400:1.
